# FASCICULE DE BREVET EUROPEEN

(11) **EP 1 100 799 B1**
(45) Date de publication et mention de la délivrance du brevet: **13.02.2002**
(21) Numéro de dépôt: 00931353.7
(22) Date de dépôt: 25.05.2000
(51) Int. Cl.: C07D 471/14

(54) **PREPARATION DE DERIVES DE LA CAMPTOTHECINE ET DE LA NOTHAPODYTINE**
VERFAHREN ZUR HERSTELLUNG VON CAMPTOTHECIN- UND NOTHAPODYNTINDERIVATEN
PREPARATION OF CAMPTOTHECIN AND NOTHAPODYTINE DERIVATIVES

(30) Priorité: 28.05.1999 FR 9906757
(43) Date de publication de la demande: 23.05.2001
(73) Titulaire: Aventis Pharma S.A., 92160 Antony (FR)
(72) Inventeur: MEKOUAR, Khalid, Montreal, Québec H1K-4C8 (CA); GENISSON, Yves, F-31400 Toulouse (FR); LEUE, Stéfanie, F-38700 La Tronche (FR); GREENE, Andrew, E., F-38410 Saint Martin d'Uriage (FR)
(74) Mandataire: Le Pennec, Magali
(86) Numéro de dépôt international: FR0001425
(87) Numéro de publication internationale: WO0073305

(56) Documents cités:
- D.L. COMINS ET AL.: "Concise synthesis of mappicine ketone and (+-)mappicine" JOURNAL OF ORGANIC CHEMISTRY., vol. 61, no. 26, 1996, pages 9623-24, XP002129454 AMERICAN CHEMICAL SOCIETY. EASTON., US ISSN: 0022-3263 cité dans la demande
- D.L. BOGER ET AL.: "Total synthesis of nothapodytine B and (-)mappicine" JOURNAL OF THE AMERICAN CHEMICAL SOCIETY., vol. 120, no. 6, 1998, pages 1218-22, XP002129455 AMERICAN CHEMICAL SOCIETY, WASHINGTON, DC., US ISSN: 0002-7863 cité dans la demande

## Description

La présente invention concerne la préparation de dérivés de la camptothécine. La présente invention concerne également la préparation de la nothapodytine, de la mappicine ou de ses dérivés.

Dans le brevet européen EP 137145, cité ici à titre de référence, ont été décrits des dérivés de camptothécine de formule générale : 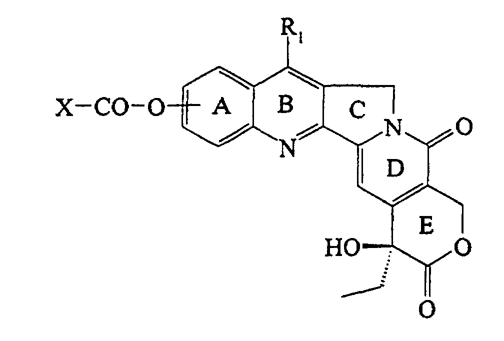 dans laquelle notamment R₁ est hydrogène, halogène ou alcoyle, X est un atome de chlore ou NR₂R₃ pour lequel R₂ et R₃ identiques ou différents peuvent représenter un atome d'hydrogène, un radical alcoyle éventuellement substitué, un carbocycle ou un hétérocycle éventuellement substitués, ou des radicaux alcoyle (éventuellement substitués) formant avec l'atome d'azote auquel ils sont attachés, un hétérocycle contenant éventuellement un autre hétéroatome choisi parmi O, S et/ou NR₄, R₄ étant un atome d'hydrogène ou un radical alcoyle et dans laquelle le groupement X-CO-O- est situé en position 9, 10 ou 11 du cycle A. Ces dérivés de camptothécine sont des agents anticancéreux, inhibiteurs de topoisomérase I, parmi lesquels l'irinotécan, pour lequel X-CO-O- est [4-(1-pipéridino)-1-pipéridino]carbonyloxy, est un principe actif particulièrement efficace sur les tumeurs solides et notamment le cancer colorectal.

Dans la demande de brevet EP 74256, citée ici à titre de référence, ont également été décrits d'autres dérivés de camptothécine qui sont mentionnés comme agents anticancéreux, notamment des dérivés de structure analogue à la structure donnée ci-dessus et dans laquelle X-CO-O- est remplacé par un radical -X'R' pour lequel X' est O ou S et R' est un atome d'hydrogène, un radical alcoyle ou acyle.

D'autres dérivés de camptothécine ont aussi été décrits par exemple dans les brevets ou demandes de brevets, cités ici à titre de référence, EP 56692, EP 88642, EP 296612, EP 321122, EP 325247, EP 540099, EP 737686, WO 9003169, WO 9637496, WO 9638146, WO 9638449, WO 9700876, US 7104894, JP 57 116015, JP 57 116074, JP 59 005188, JP 60 019790, JP 01 249777, JP 01246287, JP 91 012070 ou dans Canc. Res., 38 (1997) Abst. 1526 ou 95 (San Diego - 12-16 avril), Canc. Res., 55(3), 603-609 (1995) ou AFMC Int. Med. Chem. Symp. (1997) Abst. PB-55 (Séoul - 27 juillet-1 août).

L'irinotécan (CPT-11) et ses dérivés sont habituellement préparés à partir de la camptothécine naturelle (US 4604463 ; S. SAWADA et coll., Chem. Pharm. Bull., 39, 2574-80 (1991), Chem. Pharm. Bull., 39, 1446-54 (1991), Chem. Pharm. Bull., 39, 3183-88 (1991) et Ann. N.Y. Acad. Sci., 803, 13-28 (1996). Les étapes comportent l'introduction d'une fonction hydroxyle en 9, une alcoylation en 11 et l'introduction du radical en position 9.

La mappicine et la nothapodytine sont des produits connus, la nothapodytine est un alcaloïde naturel possèdant une activité antivirale sur les virus HSV-1, HSV-2 et HCMV.

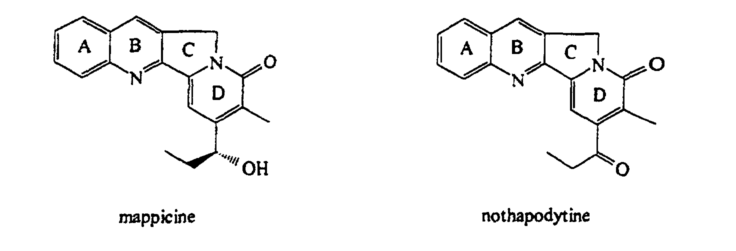

Dans la demande de brevet international WO 96/31513 a été décrite la préparation de dérivés de camptothécine et de mappicine par synthèse totale par préparation en premier lieu de l'enchaînement cyclique C-D ou C-D-E.

Tetrahedron, 53(32), 11049-60 (1997) décrit également les synthèses totales de dérivés de camptothécine dans lesquelles les cycles A-B et D-E sont préparés préalablement, ou selon un autre aspect, les enchaînements C-D-E ou A-B-C.

J. Amer. Chem. Soc., 120, 1218-1222 (1998) et J. Org. Chem, 61, 9623-24 (1996) décrivent des synthèses de nothapodytine à partir des cycles A-B avec respectivement des rendements de 17 et 30 % ; cependant ces voies de synthèse n'était pas simples à mettre en oeuvre compte tenu de la préparation des matières de départ faisant intervenir des basses températures et des problèmes d'hygienne industrielle.

Il a maintenant été trouvé, et c'est ce qui fait l'objet de la présente invention, que les dérivés de camptothécine, ainsi que la mappicine et la nothapodytine pouvaient être obtenus par une synthèse convergente à partir d'un dérivé de la 3-aminométhyl 2-bromo quinoléine et de l'acide 4-éthyl 2-méthyl hepta-2,4-diènoïque avec des résultats particulièrement intéressants.

Selon l'invention, l'acide 4-éthyl 2-méthyl hepta-2,4-diènoïque de structure : 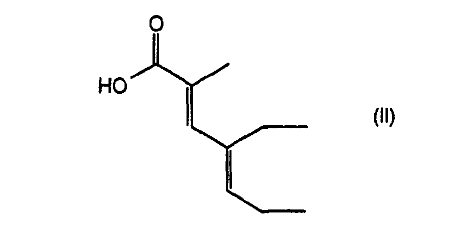 est condensé sur un dérivé de la 3-aminométhyl 2-bromo quinoléine de formule générale: 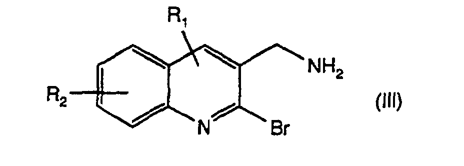 dans laquelle R₁ et R₂ peuvent être des atomes d'hydrogène ou R₁ représente un atome d'halogène ou un radical alcoyle, R₂ est un radical de structure -O-CO-X tel que cité ci-avant ou R₁ et R₂ sont définis comme dans les références citées ci-avant ou représentent des radicaux protégés ou facilement convertibles dans les radicaux R₁ et R₂ cités ci-avant, pour obtenir le dérivé de quinoléine de formule générale : 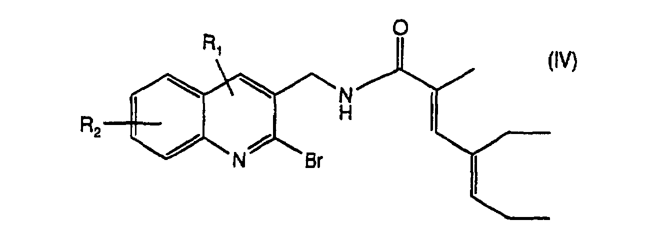 dans laquelle R₁ et R₂ sont définis comme précédemment.

La réaction s'effectue généralement selon les méthodes habituelles de condensation des acides sur les amines, notamment par action de l'acide ou d'un dérivé réactif de l'acide.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel que notamment un solvant chloré (dichlorométhane, dichloréthane, chloroforme par exemple), en présence d'un accepteur d'acide tel qu'une base organique azotée comme par exemple la pyridine, la diméthylaminopyridine, la N-méthylmorpholine ou une trialcoylamine (notamment triéthylamine, diisopropyléthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyldiimidazole ou l'éthoxy-2 éthoxycarbonyl-1 dihydro-1,2 quinoléine. De préférence on opère sous argon ou azote.

Il est entendu que, les radicaux amino, alcoylamino ou carboxy contenus dans R₁ et/ou R₂ sont de préférence préalablement protégés. Notamment, on protège selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le dérivé de quinoléine de formule générale (IV) est additionné de (2-méthoxy carbonyl vinyl) tributyl étain, en présence d'un complexe de palladium [comme par exemple le tris (dibenzylidène acétone) dipalladium] et de triphénylarsine, pour donner le dérivé de quinoléine de formule générale : 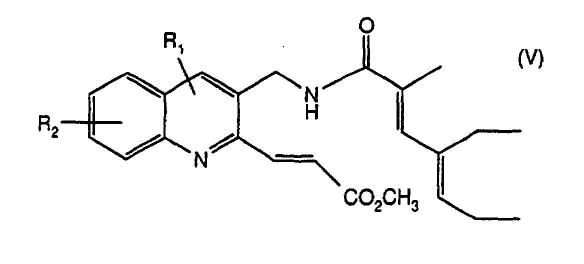 dans laquelle R₁ et R₂ sont définis comme précédemment.

La réaction s'effectue généralement dans un solvant organique tel qu'un éther (dioxane par exemple) à une température comprise entre 50 et 110°C. De préférence on opère sous argon ou azote.

Le dérivé de la quinoléine de formule générale (V) en présence de triéthylamine, est cyclisé par addition du t.butyldiméthylsilyl triflate pour donner le dérivé tétracyclique de formule générale : 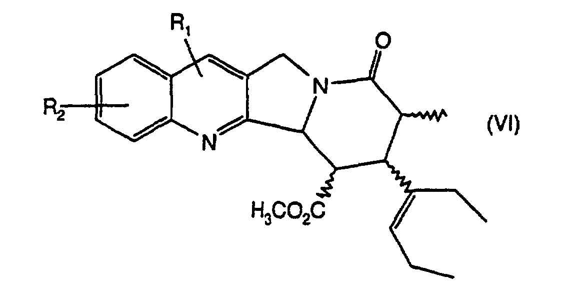 dans laquelle R₁ et R₂ sont définis comme précédemment.

La réaction est mise en oeuvre en milieu anhydre, dans un solvant organique chloré (dichlorométhane par exemple), à une température comprise entre -30 et +30°C. De préférence on opère sous argon ou sous azote.

Ce dérivé est soumis à une ozonolyse suivie du traitement par le diméthyl sulfure pour donner la cétone dérivée du composé tétracyclique de formule générale : 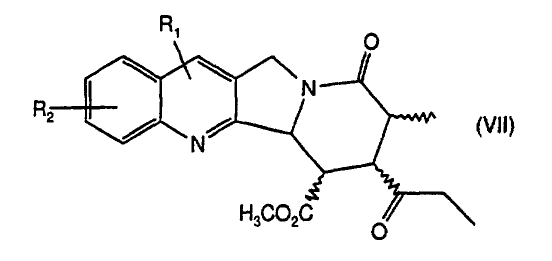 dans laquelle R₁ et R₂ sont définis comme précédemment.

L'ozonolyse est mise en oeuvre dans un mélange de solvant chloré et d'alcool (par exemple dichlorométhane/méthanol) à une température voisine de -78°C. Le produit obtenu est traité par le diméthyl sulfure à une température comprise entre -78 et 20°C.

Le dérivé tétracyclique de formule générale (VII) est saponifié puis décarboxylé dans des conditions oxydantes pour donner le dérivé de nothapodytine de formule générale : 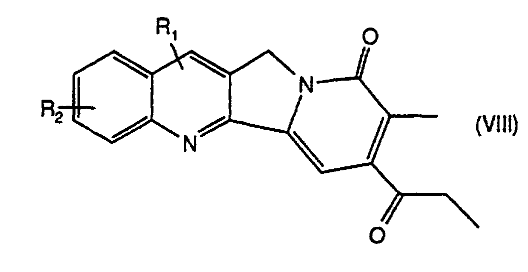 dans laquelle R₁ et R₂ sont définis comme précédemment.

La réaction est effectuée avantageusement par traitement par la soude en milieu chloro méthanolique (dichlorométhane/méthanol par exemple). Le produit ainsi obtenu est chauffé à une température comprise entre 130 et 180°C en présence de palladium sur charbon, dans de le 4-isopropyl méthylbenzène. De préférence on opère sous argon.

Le dérivé de nothapodytine de formule générale (VIII) peut être alternativement transformé en un dérivé de la mappicine par réduction de la fonction cétone par analogie avec la méthode décrite dans J. Am. Chem. Soc., 120, 1218-1222 (1998) ou éventuellement en un dérivé de la camptothécine par transformation, par exemple, en cyanohydrine puis hydrolyse en hydroxy ester par analogie avec la méthode décrite dans J. Org. Chem., 51, 5463-5465 (1986), suivie d'une oxydation à l'aide d'un oxydant tel que par exemple l'oxyde de sélénium par analogie avec la méthode décrite dans J. Am. Chem. Soc., 69, 1467 (1947) ou J. Heterocycl. Chem., 4, 163 (1967).

Le dérivé de 3-aminométhyl 2-bromo quinoléine de formule générale (III) dans laquelle R₁ et R₂ sont définis comme précédemment peut être préparée par réduction du dérivé correspondant de la 3-azidométhyl 2-bromo quinoléine de formule générale: 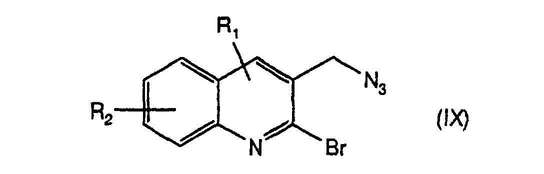 dans laquelle R₁ et R₂ sont définis comme précédemment.

La réduction est mise en oeuvre par hydrogénation catalytique en présence d'oxyde de platine en milieu alcoolique (éthanol, méthanol par exemple) à une température comprise entre 0 et 30°C.

Le dérivé de 3-azidométhyl 2-bromo quinoléine de formule générale (IX) est préparé à partir du dérivé de la 2-bromo 3-bromométhyl quinoléine de formule générale : 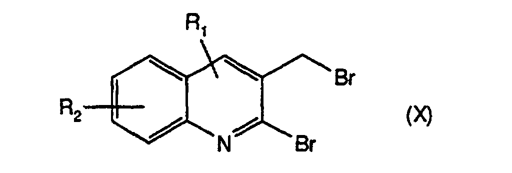 dans laquelle R₁ et R₂ sont définis comme précédemment.

La réaction s'effectue généralement par action de l'azoture de sodium dans un solvant organique comme un amide (diméthylformamide par exemple) à une température voisine de 20°C. De préférence on opère sous argon ou sous azote.

L'acide 4-éthyl 2-méthyl hepta-2,4-diènoïque de formule générale (II) peut être préparé par hydrolyse ou saponification de l'ester correspondant comme décrit ci-après dans l'exemple.

L'ester méthylique de l'acide 4-éthyl 2-méthyl hepta-2,4-diènoïque peut être obtenu selon la méthode décrite par Ei-Ichi Negishi et coll., J. Amer. Chem. Soc., 100(7), 2254-2256(1978).

Les produits obtenus selon le procédé de l'invention peuvent être purifiés selon les méthodes habituelles utilisées par l'homme de métier. Par exemple par chromatographie.

L'exemple suivant donné à titre non limitatif illustre la présente invention.

### Exemple

A une solution de 1,08 g (0,0046 mole) de 2-aminométhyl-1-bromo quinoléine en suspension dans 22 ml de CH₂Cl₂ à 5°C sont additionnés sous argon 90 mg (0,0007 mole) de diméthylaminopyridine, puis 1,04 g (0,0050 mole) de dicyclohexylcarbodiimide.

On additionne alors 0,850 g (0,0051 mole) de l'acide 4-éthyl 2-méthyl hepta-2,4-diènoïque précédemment obtenu dissous dans 6 ml de CH₂Cl₂. L'agitation est maintenue pendant 60 heures à température ambiante, le mélange est ensuite dilué avec 20 ml d'éther éthylique, le précipité ainsi formé est filtré sur 3 cm de célite.

Le solvant est évaporé sous pression réduite (2,7 kPa), le résidu ainsi obtenu est purifié sur colonne de silice (4 % CH₂Cl₂/éther) pour donner 1,57 g de (E,E)-N-(2-bromoquinolin-3-ylméthyl)-4-éthyl-2-méthyl-2,4-heptadiènamide (Rdt : 89 %).
IR (film, cm-1) ν : 3363, 2971, 2938, 1656, 1564, 1504.
RMN ¹H (CDCl₃, 200 MHz) δ : 8,18 (s, 1H) ; 7,99 (d, 1H, J = 8,6 Hz) ; 7, 80 (d, 1H, J = 7,8 Hz) ; 7,69 (ddd, 1H, J = 8,4 / 7,0 / 1,7 Hz) ; 7,54 (ddd, 1H, J = 8,0 / 7,0 / 1,4 Hz) ; 6,78 (sl, 1H) ; 6,49 (sl, 1H, NH) ; 5,36 (t, 1H, J = 7,6 Hz) ; 4,68 (2s, 2H) ; 2,14 (q, 4H, J = 7,6 Hz) ; 1,99 (d, 3H, J = 1,7 Hz) ; 0,98 (t, 3H, J= 7,6 Hz) ; 0,93 (t, 3H, J = 7,8 Hz).
RMN ¹³C (CDCl₃, 50 MHz) δ : 169,7; 147,5 ; 143,1 ; 138,1 ; 137,9 ;136,9 ; 134,3 ; 131,5 ; 130,3 ; 129,0 ; 128,1 ; 127,7 ; 127,3 ; 127,3 ; 43,4 ; 23,4 ; 21,1 ; 14,2 ; 14,1 ; 13,4.

A une solution de 1,47 g (0,0038) de (E,E)-N-(2-bromoquinolin-3-ylméthyl)-4-éthyl-2-méthyl-2,4-heptadiènamide dissous dans 59 ml de dioxane sec, on additionne sous argon et à température ambiante 120 mg (0,00013 mole) de tris (dibenzylidèneacétone) dipalladium ; le mélange réactionnel est agité pendant 15 minutes, avant d'additionner 162 mg (0,0005 mole) de triphényl arsine.

Le mélange se décolore rapidement, après 30 minutes d'agitation, on additionne 2,28 g (0,0061 mole) de (E)-(2-méthoxycarbonylvinyl) tributyl étain dissous dans 10 ml de dioxane, suivie de quelques cristaux de 2,6-di-tert-butyl-4-méthylphénol (BHT).

Le mélange réactionnel est alors porté à 80°C pendant 7 heures, il est ensuite ramené à température ambiante et traité avec quelques gouttes d'une solution de H₂O / KF (2/1).

Après 10 minutes d'agitation, le mélange est repris avec 25 ml de H₂O et extrait avec 3 fois 60 ml de CH₂Cl₂.

La phase organique est séchée sur MgSO₄, filtrée, le solvant est évaporé à sec sous pression réduite (2,7 kPa). Le résidu ainsi obtenu est purifié sur colonne de silice (gradient : 100 % CH₂Cl₂; 4 % ; 7 % ; 15 % ; 30 % éther/CH₂Cl₂) pour donner 1,10 g de l'ester méthylique de l'acide (E)-3-(3-(((E,E)-4-éthyl-2-méthyl-2,4-heptadiènamido)-méthyl)quinolin-2-yl) acrylique. PF : 119-121°C (Rdt : 74 %).
IR (film, cm⁻¹) ν : 3371, 3053, 2987, 1716, 1656.
RMN ¹H (CDCl₃, 200 MHz) δ : 8,12 (s, 1H) ; 8,08-7,96 (m, 1H); 7, 77 (d, 1H, J = 7,8 Hz) ; 7,69 (t, 1H, J = 7,2 Hz) ; 7,64 (ABq, 2H, δₐ = 8,03, δ_{b} = 7,25, J_{ab} = 15,2 Hz ; 7,51 (t, 1H, J = 7,2 Hz) ; 6,74 (sl, 1H) ; 6,13 (sl, NH) ; 5,36 (t, 1H, J = 7,6 Hz) ; 4,79 (2 s, 2H) ; 3,83 (s, 3H) ; 2,19-2,06 (m, 4H) ; 1,98 (s, 3H) ; 1,02 (t, 3H, J = 7,6 Hz) ; 0,92 (t, 3H, J = 7,9 Hz).
RMN ¹³C (CDCl₃, 50 MHz) δ : 169,8 ; 167,0 ; 151,1; 147,3 ; 139,1 ; 137,6 ; 136,9 ; 136,3 ; 134,3 ; 130,2 ; 130,0 ; 129,5 ; 129,2 ; 128,1 ; 127,4 ; 127,3 ; 125,0 ; 51,9 ; 41,0 ; 23,4 ; 21,2 ; 14,2 ; 14,1 ; 13,4.

0,340 g (0,00087 mole) de l'ester méthylique de l'acide (E)-3-(3-(((E,E)-4-éthyl-2-méthyl-2,4-heptadiènamido)-méthyl)quinolin-2-yl) acrylique est dissous dans 9 ml de CH₂Cl₂ sec, avant d'additionner sous argon 0,6 ml (0,0043 mole) de triéthylamine.

Le mélange réactionnel est agité et refroidi à 10°C avant d'additionner sous argon 0,590 ml (0,0026 mole) de t.butyldiméthylsilyl triflate.

L'agitation est maintenue pendant 3 heures 30, puis le mélange réactionnel est traité avec 8 ml de H₂O, et il est extrait avec 3 fois 10 ml de CH₂Cl₂. La phase organique est séchée sur MgSO₄, elle est ensuite filtrée, le solvant est évaporé sous pression réduite (2,7 kPa), le résidu obtenu est purifié sur colonne de silice (gradient: éther / CH₂Cl₂ : 4 à 10 %) pour donner 0,240 g de diastéréoisomère majoritaire, suivie de 0,045 g de diastéréoisomères minoritaires, soit un rendement global de 84 % pour la préparation de l'ester méthylique de l'acide 7-(1-éthyl-1-butényl)-8-méthyl-5b,6,7,8-tétrahydro-11H-indolizino[1,2-b]quinolin-9-one-6-carboxylique.

Pour diastéréoisomère majoritaire :
IR (film, cm-1) ν : 3061, 3053, 2971, 2930, 2873, 1739, 1649, 1576, 1501, 1461, 1436, 1406.
RMN ¹H (CDCl₃, 300 MHz) δ : 8,05 (s, 1H); 7, 98 (d, 1H, J = 8,5 Hz) ; 7,79 (d, 1H, J = 8,0 Hz) ; 7,66 (ddd, 1H, J = 8,5 / 6,9 / 1,5 Hz) ; 7,52 (ddd, 1H, J = 8,1 / 7,0 / 1,3 Hz) ; 5,22-5,08 (m, 2H) ; 4,93 (ABq, 2H, δₐ = 5,12, δ_{b} = 4,73, J_{ab} = 16,4 Hz) ; 3,88 (s, 3H) ; 3,16-2,76 (m, 3H) ; 2,10-1,73 (m, 4H) ; 1,10 (d, 3H, J = 7,2 Hz) ; 0,93 (t, 3H, J = 7,0 Hz) ; 0,90 (t, 3H, J = 7,6 Hz).
RMN ¹³C (CDCl₃, 50 MHz) δ : 173,4 ; 172,7 ; 160,5 ; 149,0 ; 138,0 ; 130,7 ; 130,2 ; 129,5 ; 129,3 ; 127,8 ; 127,7 ; 127,6 ; 126,7 ; 63,0 ; 51,9 ; 49,2 ; 48,4 ; 48,1 ; 38,5 ; 23,5 ; 21,0 ; 14,4 ; 13,5 ; 13,5.

Dans un bicole de 50 ml, on dissout sous argon 0,100 g (0,00025 mole) de l'ester méthylique de l'acide 7-(1-éthyl-1-butényl)-8-méthyl-5b,6,7,8-tétrahydro-11H-indolizino[1,2-b]quinolin-9-one-6-carboxylique (diastéréoisomère majoritaire) dans un mélange de 12 ml de CH₂Cl₂ et de 2 ml de MeOH.

Le mélange réactionnel est refroidi à -78°C et le courant d'argon est remplacé par un courant d'oxygène. L'ozonolyseur est branché, pendant 2 fois 90 secondes, puis l'excès d'ozone est purgé à l'oxygène pendant 15 minutes. 2 ml de diméthyl sulfure sont ajoutés à -78°C, en laissant la température remonter progressivement à 20°C pendant une nuit.

Le solvant est évaporé à sec, le résidu ainsi obtenu est repris avec 10 ml de H₂O, et extrait avec CH₂Cl₂. La phase organique est séchée, puis filtrée, le solvant est évaporé sous pression réduite (2,7 kPa) et le résidu ainsi obtenu est purifié sur colonne de silice (15 % éther/CH₂Cl₂) pour donner 56 mg de l'ester méthylique de l'acide 8-méthyl-7-propionyl-5b,6,7,8-tétrahydro-11H-indolizino[1,2-b]quinolin-9-one-6-carboxylique.
PF : 178-181 °C, soit 60 % de rendement.
IR (film, cm⁻¹) ν : 2922, 2848, 1730, 1657.
RMN ¹H (CDCl₃, 200 MHz) δ : 8,04 (s, 1H) ; 7, 96 (d, 1H, J = 8,2 Hz) ; 7,79 (dd, 1H, J = 8,1 / 1,4 Hz) ; 7,66 (ddd, 1H, J = 8,4 / 6,8 / 1,4 Hz) ; 7,52 (ddd, 1H, J = 8,1 / 6,8 / 1,4 Hz) ; 5,3-5,08 (m, 2H) ; 4,93 (ABq, 2H, δₐ = 5,15, δ_{b} = 4,72, J_{ab} = 15,9 Hz) ; 3,90 (s, 3H) ; 3,60-3,44 (m, 1H) ; 3,10-2,92 (m, 1H) ; 2,47 (qd, 2H, J = 7,2 / 1,4 Hz) ; 1,16 (d, 3H, J = 7,2 Hz) ; 1,01 (t, 3H, J = 7,2 Hz).
RMN ¹³C (CDCl₃, 50 MHz) δ : 209,5 ; 173,1 ; 170,5 ; 160,0 ; 149,1 ; 130,2 ; 129,5 ; 129,4 ; 127,8 ; 127,6 ; 127,5 ; 126,8 ; 62,2 ; 53,5 ; 52,4 ; 48,6 ; 45,3 ; 37,1 ; 36,1 ; 13,8 ; 7,3.

A 0,100 g (0,00027 mole) d'une solution de céto-ester dans 4 ml de CH₂Cl₂, est additionné 0,47 ml d'une solution de NaOH 2 N, suivie de 0,9 ml de MeOH.

Le mélange réactionnel est agité pendant 65 heures à température ambiante, il est ensuite dilué avec 8 ml de H₂O, puis acidifié jusqu'à pH = 2-2,5 avec une solution de HCl concentrée, et extrait avec CH₂Cl₂. La phase organique est séchée, puis filtrée et le solvant est évaporé sous pression réduite (2,7 kPa).

Le produit brut ainsi obtenu est mis en suspension dans 4 ml de 4-isopropyl méthylbenzène, 75 mg de Pd/C 10 % sont ensuite ajoutés sous argon, et le mélange réactionnel est chauffé à reflux pendant 1 heure 30.

Après refroidissement, le mélange est purifié directement sur une courte colonne de silice (100 % CH₂Cl₂ ; 2 % MeOH/CH₂Cl₂) pour donner 0,071 g du produit désiré qui est lavé à froid avec 2 fois 1 ml de MeOH glacé pour donner 0,050 g de 8-méthyl-7-propionyl-11H-indolizino[1,2-b]quinolin-9-one (nothapodytine). PF : 236-237°C, soit un rendement global de 60 %.
IR (KBr, cm-1) ν : 3094 ; 2988 ; 2939 ; 1703 ; 1651 ; 1600 ; 1443 ; 1378 ; 1226 ; 1184; 1143 ; 932 ; 838 ; 762.
RMN ¹H (CDCl₃, 300 MHz) δ: 8,34 (s, 1H) ; 8,17 (d, 1H, J = 8,6 Hz) ; 7,90 (d, 1H, J = 7,9 Hz) ; 7,79 (ddd, 1H, J=8,4/6,9/1, 6 Hz); 7,62 (ddd, 1H, J = 8,1 / 6,8 /1,2 Hz) ; 7,23 (s, 1H) ; 5,27 (2s, 2H) ; 2,89 (q, 2H, J = 7,2 Hz) ; 2,28 (s, 3H) ; 1,22 (t, 3H, J = 7,2 Hz).
RMN¹³C (CDCl₃, 75,5 MHz) δ : 205,5 ; 161,7 ; 152,8 ; 148,8 ; 148,1 ; 143,3 ; 131,0 ; 130,4 ; 129,5 ; 128,5 ; 128,1 ; 128,0 ; 127,7 ; 127,0 ; 97,8 ; 50,2 ; 36,0 ; 13,6 ; 7,7.

La 3-azidométhyl 2-bromo quinoléine peut être préparée de la manière suivante :

A une solution de 3,69 g (0,0123 mole) du 2-bromo-3-bromométhyle quinoléine dissous dans 65 ml de diméthylformamide sec, sont additionnés sous argon et à température ambiante 4,0 g (0,062 mole) d'azoture de sodium.

Le mélange réactionnel est agité alors pendant 17 heures, avant d'être repris avec 60 ml d'eau et extrait avec 3 fois 80 ml de CH₂Cl₂.

La phase organique est recueillie, puis elle est lavée avec 2 fois 30 ml de H₂O, elle est ensuite séchée sur MgSO₄, filtrée, le solvant est évaporé sous pression réduite (2,7 kPa), le résidu ainsi obtenu est purifié sur colonne de silice (5†% AcOEt/cyclohexane) pour donner 2,63 g. PF : 54-56°C (Rdt : 82 %).
IR (nujol, cm-1) ν : 1706, 1461, 1379.
RMN ¹H (CDCl₃, 200 MHz) δ : 8,06 (s, 1H) ; 7,99 (dd, 1H, J = 8,2 / 1,4 Hz) ; 7,78 (dd, 1H, J = 7,9 / 1,4 Hz) ; 7,68 (ddd, 1H, J = 8,2 / 7,0 / 1,4 Hz) ; 7,55 (ddd, 1H, J = 7,9 / 7,0 /1,4 Hz) ; 4,6 (s, 2H).
RMN ¹³C (CDCl₃, 50 MHz) δ : 147,7 ; 142,2 ; 136,8 ; 130,7 ; 129,4 ; 128,3 ; 127,6 ; 127,5 ; 127,0 ; 53,7.

La 3-aminométhyl 2-bromo quinoléine peut être préparée de la manière suivante :

1,02 g (0,0039 mole) de l'azide sont dissous dans 110 ml d'éthanol à 95 %, le mélange est purgé à l'argon, avant d'additionner 30 mg d'oxyde de platine hydraté.

L'argon est purgé 3 fois à l'hydrogène, puis le mélange est agité en présence d'hydrogène pendant 2 heures à 20°C.

Le mélange réactionnel est purgé à l'argon et ensuite filtré sur 3 cm de célite. le filtrat est concentré sous pression réduite (2,7 kPa) pour donner 0,863 g soit 94 % de rendement.
IR (KBr, cm-1) ν : 3338 ; 2922 ; 1614 ; 1586 ; 1556 ; 1485 ; 1449 ; 1391 ; 1317.
RMN ¹H (CDCl₃, 200 MHz) δ : 8,11 (s, 1H) ; 7,99 (d, 1H, J = 8,2 Hz) ; 7,78 (d, 1H, J = 7,9 / 1,4 Hz) ; 7,66 (ddd, 1H, J = 8,2 / 7,0/ 1,4 Hz) ; 7,53 (ddd, 1H, J = 7,9 / 7,0 / 1,4 Hz) ; 4,04 (s, 2H).
RMN ¹³C (CDCl₃, 50 Mhz) δ : 147,4, 143,5 ; 136,2 ; 135,6 ; 129,9 ; 128,2 ; 127,6 ; 127,4 ; 127,2 ; 45,8.

L'ester méthylique de l'acide 4-éthyl 2-méthyl hepta-2,4-diènoïque peut être obtenu selon la méthode décrite par Ei-Ichi Negishi, Nobuhisa Okukado, Anthony O. King, David E. Van Horn, Barry I. Spiegel, J. Amer. Chem. Soc., 100(7), 2254-2256 (1978).
A) Préparation du vinyl zirconium :
   A une solution de 20 ml de tétrahydrofurane est additionné sous argon et à température ambiante 5,0 g (0,0194 mole) de l'hydrochlorure de zirconocène, suivie de 2,20 ml (0,01936 mole) de 3-hexyne.
   Le mélange réactionnel devient homogène, rouge clair au bout d'une heure, l'agitation est maintenue pendant 2 heures supplémentaires.
B) A une solution de 20 ml de tétrahydrofurane, est additionné sous argon et à une température ambiante 5 % mole de tétrakis-triphénylphosphine palladium, suivie de l'addition goutte à goutte de 3,46 g (0,01933 mole) de (E)-2-bromométhacrylate de méthyle dissous dans 5 ml de tétrahydrofurane, immédiatement suivie de l'addition de 2,60 g (0,0191 mole) de chlorure de zinc.

* (E)-2-Bromométhacrylate de méthyle préparé selon Bull. Soc. Chim. Fr, N° 3-4, 487-492 (1976).

Après 24 heures d'agitation, on ajoute 50 ml de H₂O, on extrait par 4 fois 50 ml de CH₂Cl₂, la phase organique est ensuite séchée sur MgSO₄ et filtrée. Après concentration sous pression réduite (2,7 kPa) à une température inférieur à 20°C, le solide ainsi obtenu est extrait au pentane : 3 fois 30 ml, la phase liquide est filtrée sur coton, puis le pentane est évaporé sous pression réduite (2,7 kPa) à température inférieur à 20°C et le résidu ainsi obtenu est chromatographié sur colonne de silice (3 % AcOEt/Cyclohexane) pour fournir 2,12 g de l'ester méthylique désiré (Rdt : 61 %).
IR (film, cm-1) ν : 2971, 2938, 2865, 1706, 1632, 1257.
RMN ¹H (CDCl₃, 200 MHz) δ : 7,06 (sl, 1H); 5,45 (t, 1H, J = 8,0 Hz); 3,72 (s, 3H) ; 2,23-2,04 (m, 4H) ; 1,95 (d, 3H, J = 1,4 Hz) ; 0,99 (t, 3H, J = 7,6 Hz) ; 0,93 (t, 3H, J†= 7,6 Hz).
RMN ¹³C (CDCl₃, 50 MHz) δ : 169,4 ; 142,0 ; 137,2 ; 135,5 ; 126,0 ; 51,7 ; 23,2 ; 21,2; 14,1 ; 13,8; 13,4.

L'acide 4-éthyl 2-méthyl hepta-2,4-diènoïque peut être obtenu de la manière suivante:

A une solution de 1,58 g (0,0087 mole) de l'ester dissous dans 17 ml de MeOH, est additionné 10,8 ml (0,0108 mole) d'une solution de soude IN.

Le mélange réactionnel est alors porté à reflux pendant 30 minutes, il est ensuite dilué à température ambiante avec 20 ml d'une solution saturée de NaHCO₃, puis extrait avec 10 ml d'éther éthylique.

La phase aqueuse est acidifiée à pH acide avec une solution concentrée de HCl à 0°C, elle est ensuite extraite avec 3 fois 15 ml de CH₂Cl₂. La phase organique est séchée sur MgSO₄, filtrée, le solvant évaporé sous pression réduite (2,7 kPa) à froid pour donner 1,20 g de l'acide désiré (Rdt : 82 %).
IR (film, cm-¹) ν: 3249, 2963, 2930, 2873, 1678, 1627, 1415.
RMN ¹H (CDCl₃, 200 Mhz), δ : 10,4 (sl, 1H) ; 7,2 (sl, 1H) ; 5,53 (t, 1H, J = 7,2 Hz) ; 2,26-2,06 (m, 4H) ; 1,94 (d, 3H, J = 1,6 Hz) ; 1,01 (t, 3H, J = 7,2 Hz) ; 0,96 (t, 3H, J = 7,8 Hz).

Les dérivés de la camptothécine sont habituellement administrés par voie injectable, plus particulièrement par voie intraveineuse sous forme de solution stérile ou d'une émulsion. Les dérivés de la camptothécine peuvent être également administrés par voie orale, sous forme de compositions solides ou liquides.

Lorsque le dérivé de camptothécine est administré par voie intraveineuse, ces compositions peuvent également contenir des adjuvants, en particulier des agents mouillants, isotonisants, émulsifiants, dispersants et stabilisants. L'irinotécan (CPT-11) est notamment administré en solution dans un milieu pour injection intraveineuse, à des doses comprises entre 175 à 500 mg/m².

## Revendications

1. Un procédé de préparation de dérivés de la nothapodytine ou de dérivés de la mappicine ou de la camptothécine **caractérisé en ce que**
a) on fait agir l'acide 4-éthyl 2-méthyl hepta-2,4-diènoïque sur un dérivé de la 3-aminométhyl 2-bromo quinoléine de formule générale : 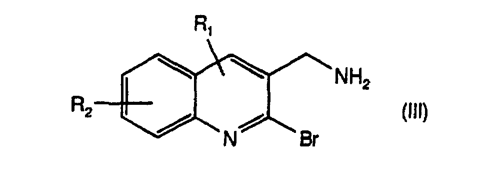 dans laquelle R₁ et R₂ peuvent être des atomes d'hydrogène ou R₁ représente un atome d'halogène ou un radical alcoyle, R₂ est un radical de structure -O-CO-X tel que défini pour les dérivés de la camptothécine ou R₁ et R₂ sont définis comme pour les dérivés de camptothécine connus ou représentent des radicaux protégés ou facilement convertibles dans les radicaux R₁ et R₂ cités ci-avant, pour obtenir le dérivé de quinoléine de formule générale : 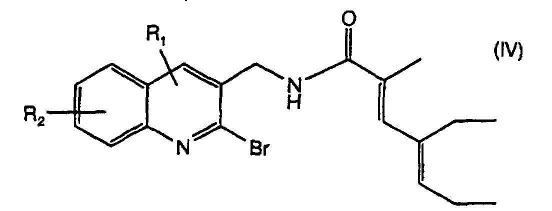 dans laquelle R₁ et R₂ sont définis comme précédemment ;
b) au dérivé de quinoléine obtenu, on additionne du (2-méthoxy carbonyl vinyl) tributyl étain en présence d'un complexe de palladium et de triphénylarsine, pour donner le dérivé de quinoléine de formule générale: 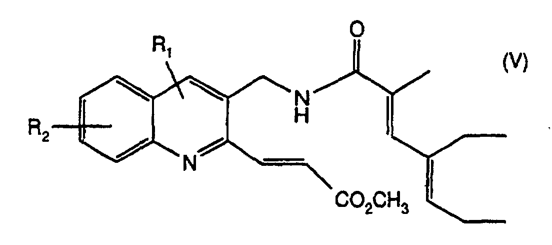 dans laquelle R₁ et R₂ sont définis comme précédemment ;
c) on cyclise le dérivé de la quinoléine obtenu pour donner le dérivé tétracyclique de formule générale : 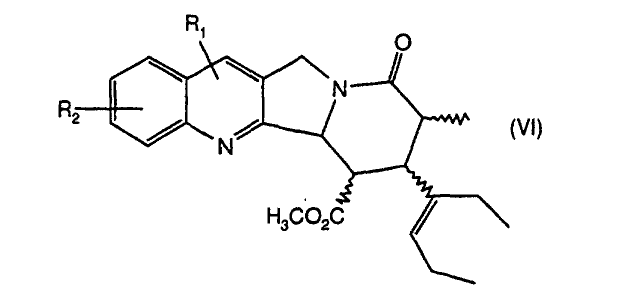 dans laquelle R₁ et R₂ sont définis comme précédemment, puis soumet ce dérivé à une ozonolyse suivie du traitement par le diméthyl sulfure pour donner la cétone dérivée du composé tétracyclique de formule générale : 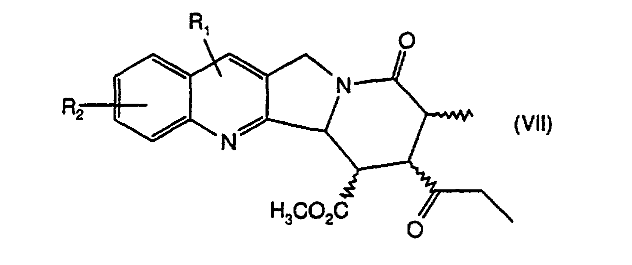 dans laquelle R₁ et R₂ sont définis comme précédemment ;
d) on saponifie puis décarboxyle dans des conditions oxydantes le dérivé tétracyclique ainsi obtenu pour donner le dérivé de nothapodytine de formule générale : 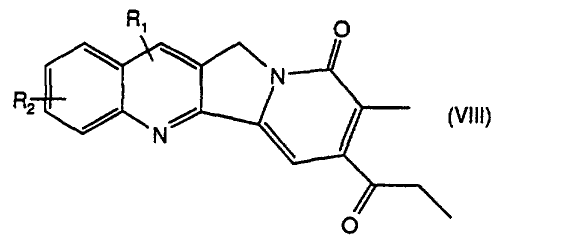
dans laquelle R₁ et R₂ sont définis comme précédemment, puis transforme éventuellement le dérivé obtenu en un dérivé de la camptothécine ou en un dérivé de la mappicine.

2. Un procédé selon la revendication 1 **caractérisé en ce que** l'étape de cyclisation (c) est mise en oeuvre en présence de triéthylamine et de t.butyldiméthylsilyl triflate.

3. Procédé de préparation de la nothapodytine ou de ses dérivés **caractérisé en ce que**
a) on cyclise le composé de formule (V) obtenu à l'étape b) de la revendication 1
b) puis on soumet le produit obtenu à l'étape a) à une ozonolyse suivie du traitement par le diméthylsulfure
c) puis on saponifie et décarboxyle le produit obtenu à l'étape b).

## Patentansprüche

1. Verfahren zur Herstellung von Derivaten von Nothapodytin, Mappicin oder Camptothecin, **dadurch gekennzeichnet, daß** man
a) 4-Ethyl-2-methyl-hepta-2,4-diensäure mit einem Derivat von 3-Aminomethyl-2-brom-chinolin der allgemeinen Formel (III) 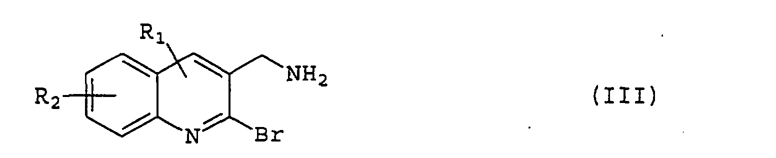 zur Reaktion bringt, in der R₁ und R₂ Wasserstoffatome sein können oder R₁ ein Halogenatom oder ein Rest Alkyl ist, R₂ einen Rest der Struktur -O-CO-X wie bei den Derivaten von Camptothecin definiert darstellt oder R₁ und R₂ wie bei den bekannten Derivaten von Camptothecin definiert sind oder geschützte Reste oder einfach in die wie oben genannten Reste R₁ und R₂ überführbare Reste bedeuten, um das Chinolin-Derivat der allgemeinen Formel (IV) 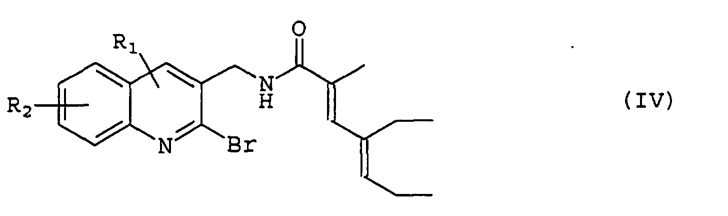 zu erhalten, in der R₁ und R₂ wie vorstehend definiert sind;
b) zu dem erhaltenen Chinolin-Derivat (2-Methoxy-carbonyl-vinyl)-tributyl-zinn in Anwesenheit eines Komplexes von Palladium und Triphenylarsin gibt, um das Chinolin-Derivat der allgemeinen Formel (V) 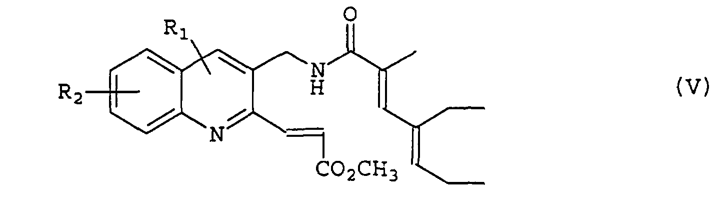 zu erhalten, in der R₁ und R₂ wie oben definiert sind;
c) das erhaltene Chinolin-Derivat cyclisiert, um das tetracyclische Derivat der allgemeinen Formel (VI) 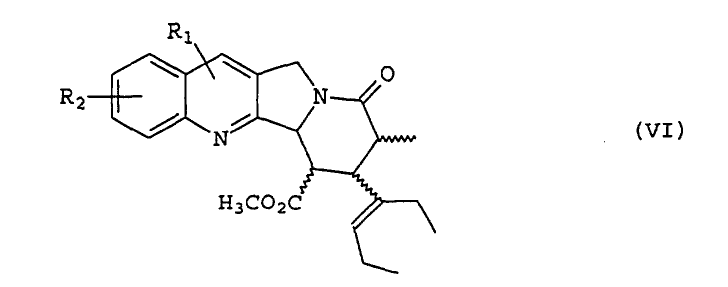 zu erhalten, in der R₁ und R₂ wie vorstehend definiert sind, wobei man dieses Derivat anschließend einer Ozonolyse, gefolgt von einer Behandlung mit Dimethylsulfid unterzieht, um das Keton-Derivat der tetracyclischen Verbindung der allgemeinen Formel (VII) 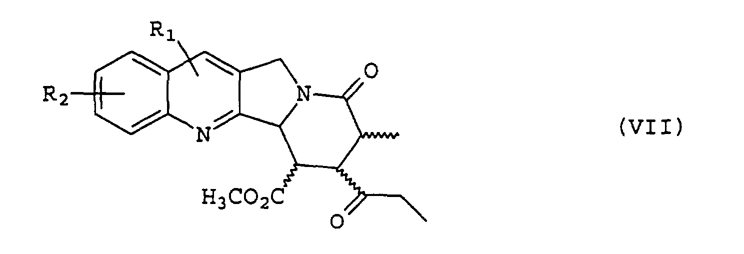 zu erhalten, in der R₁ und R₂ wie vorstehend definiert sind;
d) das auf diese Weise erhaltene tetracyclische Derivat verseift und anschließend unter oxidierenden Bedingungen decarboxyliert, um das Derivat von Nothapodytin der allgemeinen Formel (VIII) 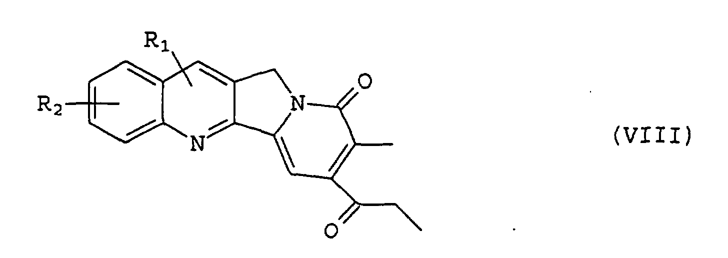 zu erhalten, in der R₁ und R₂ wie vorstehend definiert sind, und man danach das erhaltene Derivat gegebenenfalls in ein Derivat von Camptothecin oder in ein Derivat von Mappicin umwandelt.

2. Verfahren nach Anspruch 1, **dadurch gekennzeichnet, daß** die Stufe der Cyclisierung (c) in Anwesenheit von Triethylamin und tert.-Butyldimethylsilyl-triflat durchgeführt wird.

3. Verfahren zur Herstellung von Nothapodytin oder seinen Derivaten, **dadurch gekennzeichnet, daß** man
a) die in der Stufe b) von Anspruch 1 erhaltene Verbindung der Formel (V) cyclisiert,
b) das in der Stufe a) erhaltene Produkt einer Ozonolyse, gefolgt von einer Behandlung mit Dimethylsulfid unterzieht,
c) anschließend das in Stufe b) erhaltene Produkt verseift und decarboxyliert.

## Claims

1. Process for preparing derivatives of nothapodytine or derivatives of mappicine or of camptothecin, **characterized in that**
a) 4-ethyl-2-methylhepta-2,4-dienoic acid is reacted on a 3-aminomethyl-2-bromoquinoline derivative of general formula: 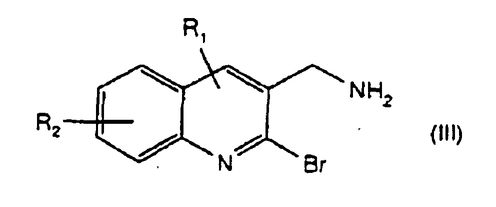 in which R₁ and R₂ may be hydrogen atoms or R₁ represents a halogen atom or an alkyl radical, R₂ is a radical having the structure -O-CO-X as defined above for the derivatives of camptothecin or R₁ and R₂ are as defined for the known derivatives of camptothecin or represent radicals which are protected or which can be easily converted to the radicals R₁ and R₂ cited above, to give the quinoline derivative of general formula: 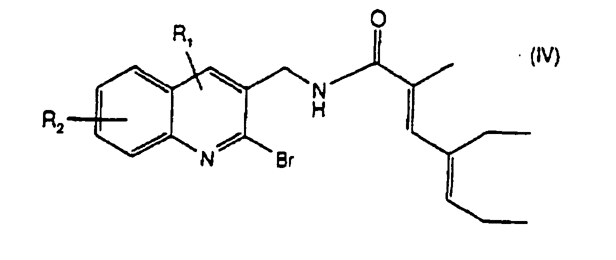 in which R₁ and R₂ are as defined above;
b) to the quinoline derivative obtained, (2-methoxycarbonylvinyl)tributyltin is added in the presence of a complex of palladium and triphenylarsine, to give the quinoline derivative of general formula: 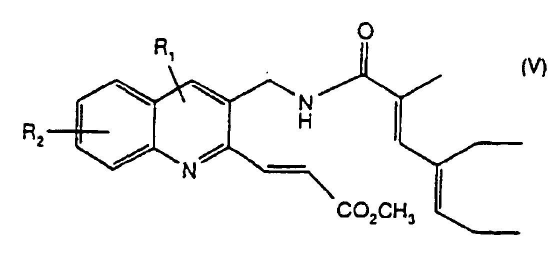 in which R₁ and R₂ are as defined above;
c) the quinoline derivative obtained is cyclized to give the tetracyclic derivative of general formula: 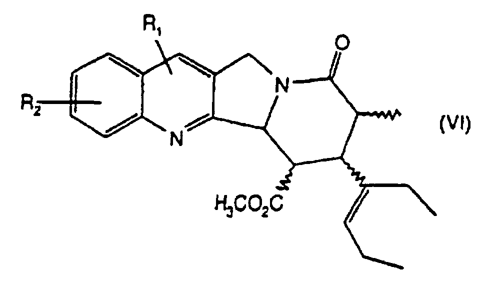 in which R₁ and R₂ are as defined above, then this derivative is subjected to ozonolysis followed by treatment with dimethyl sulphide to give the ketone derived from the tetracyclic compound of general formula: 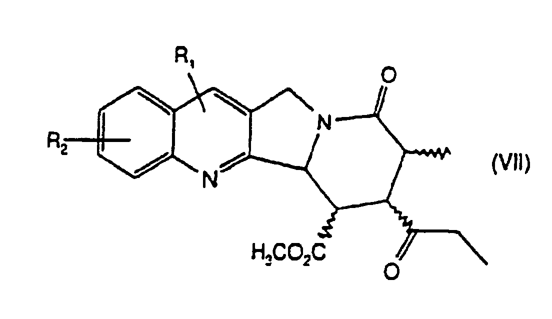 in which R₁ and R₂ are as defined above;
d) the tetracyclic derivative thus obtained is saponified and then decarboxylated under oxidizing conditions to give the nothapodytine derivative of general formula: 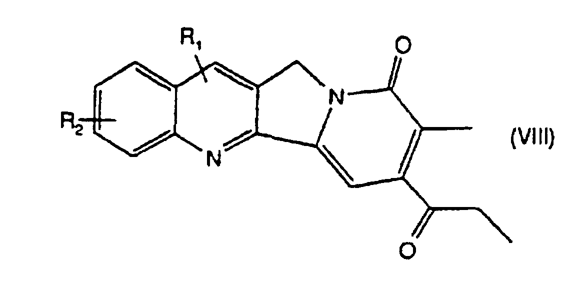 in which R₁ and R₂ are as defined above, and then the derivative obtained is optionally converted to a camptothecin derivative or to a mappicine derivative.

2. Process according to Claim 1, **characterized in that** the cyclization step (c) is carried out in the presence of triethylamine and of t-butyldimethylsilyl triflate.

3. Process for preparing nothapodytine or its derivatives, **characterized in that**
a) the compound of formula (V) obtained in step b) of Claim 1 is cyclized
b) then the product obtained in step a) is subjected to ozonolysis followed by treatment with dimethyl sulphide
c) then the product obtained in step b) is saponified and decarboxylated.
